# EUROPÄISCHE PATENTSCHRIFT

(11) **EP 1 144 416 B1**
(45) Veröffentlichungstag und Bekanntmachung des Hinweises auf die Patenterteilung: **02.03.2005**
(21) Anmeldenummer: 00972903.9
(22) Anmeldetag: 09.11.2000
(51) Int. Cl.: C07D 493/04, C07D 495/04, C07D 491/04, C09K 9/02, C08K 5/15, G02B 5/23

(54) **HETEROCYCLISCH ANNELLIERTE INDENOCHROMENDERIVATE**
HETEROCYCLICALLY ANELLATED INDENOCHROMENE DERIVATIVES
DERIVES INDENOCHROMES HETEROCYCLIQUEMENT ANNELES

(30) Priorität: 10.11.1999 DE 19954170; 11.11.1999 DE 19954435
(43) Veröffentlichungstag der Anmeldung: 17.10.2001
(73) Patentinhaber: Rodenstock GmbH, 80469 München (DE)
(72) Erfinder: MANN, Claudia, 80634 München (DE); MELZIG, Manfred, 82234 Wessling (DE); WEIGAND, Udo, 80638 München (DE)
(74) Vertreter: Müller-Boré & Partner Patentanwälte
(86) Internationale Anmeldenummer: PCT/EP2000/011078
(87) Internationale Veröffentlichungsnummer: WO 2001/034609

(56) Entgegenhaltungen:
- EP-A- 0 987 260
- WO-A-97/48762
- WO-A-98/28289
- WO-A-99/15518
- WO-A-99/23071
- US-A- 3 567 605
- US-A- 5 631 720
- US-A- 5 645 767
- US-A- 5 698 141
- US-A- 5 869 658
- US-A- 5 961 892

## Beschreibung

Die vorliegende Erfindung betrifft photochrome Indenochromenderivate sowie deren Verwendung in Kunststoffen aller Art, insbesondere für ophthalmische Zwecke. Insbesondere betrifft die vorliegende Erfindung spezifische photochrome, heterocyclisch annellierte Indeno[1,2-h]chromen-Derivate.

Es sind verschiedene Farbstoffklassen bekannt, die bei Bestrahlung mit Licht bestimmter Wellenlängen, insbesondere Sonnenstrahlen, reversibel ihre Farbe wechseln. Dies rührt daher, daß diese Farbstoffmoleküle durch Energiezufuhr in Form von Licht in einen angeregten farbigen Zustand übergehen, den sie bei Unterbrechung der Energiezufuhr wieder verlassen, wodurch sie in ihren farblosen oder zumindest kaum gefärbten Normalzustand zurückkehren. Zu diesen photochromen Farbstoffen gehören beispielsweise die Naphthopyrane, die im Stand der Technik mit verschiedenen Substituenten bereits beschrieben wurden.

Pyrane, speziell Naphthopyrane und von diesen abgeleitete größere Ringsysteme, sind photochrome Verbindungen, die bis heute Gegenstand intensiver Untersuchungen sind. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen.

Heterocyclisch annellierte Benzopyrane sind beispielsweise aus US-A-5,631,720 bekannt, wobei relativ geringfügige Strukturveränderungen am Heterozyklus einen enormen Einfluß (etwa Faktor 5) auf die Aufhellgeschwindigkeit haben. Die in US-A-5,631,720 beschriebenen Verbindungen weisen jedoch den Nachteil auf, daß sie nur den Bereich von etwa 430-450 nm (Absorptionsmaximum der angeregten Form) abdecken, sich also nur orange bis rote Farbtöne erzielen lassen.

In WO 98/28289 werden Verbindungen beschrieben, die zwar stumpfe, rote bis rotviolette Farbtöne liefern, der bathochrome Einfluß der Substitution gegenüber 2 Methylgruppen ist jedoch mit 25 nm gering. Auf die Aufhellkinetik ist der Einfluß negativ, die Aufhellgeschwindigkeit ist in allen Fällen etwas verlangsamt. Die vorgegebene Struktur läßt nur Substitutionen zu, mit denen die Aufhellgeschwindigkeit kaum beeinflußt werden kann. Fluoreno- und Naphthopyrane, die rotviolett bis grüne Farbtöne ergeben, sind beispielsweise aus WO 99/15518 und DE 198 24 278 bzw. DE 199 02 771 bekannt. Sie zeichnen sich durch ausgezeichnete Langzeitstabilität und gute Temperaturstabilität (geringe Abhängigkeit der Eindunkelungstiefe von der Temperatur) aus. Die darin beschriebenen Substitutionsmöglichkeiten lassen jedoch bezüglich der Aufhellgeschwindigkeit nur einen vergleichsweise geringen Spielraum zu.

Die Einführung einer heterocyclischen Komponente durch Ersatz des zentralen C-Atoms der Fluorenstruktur durch N-R, wie in WO 99/23071 vorgeschlagen, führt zu Verbindungen, deren Absorptionsmaximum in der nicht-angeregten Form um bzw. oberhalb 400 nm, also deutlich im Sichtbaren liegt. Da sich die Absorption über das Maximum hinaus noch weit in den violetten und blauen Spektralbereich erstreckt, werden jedoch Verbindungen erhalten, die im nicht-angeregten Zustand stark gelb gefärbt sind. Sie sind daher für Anwendungen in Brillengläsern ungeeignet.

Ebenso nachteilig ist die Einführung einer heterocyclischen Einheit an den beiden Benzolringen der Indenonaphthopyranstruktur, wie in US-A-5,698,141 und US-A-5,723,072 beschrieben. Die Eindunkelungstiefe dieser Verbindungen (meist als colorability A₀ angegeben) ist im Vergleich zu den Verbindungen von US-A-5,631,720 gering. Zudem werden hierbei Verbindungen mit sehr vielen annellierten Ringen erhalten, die meist bereits in dem nicht-angeregten Zustand deutlich im Sichtbaren absorbieren, d.h. die Verbindungen sind im nicht-angeregten Zustand gelb. Insofern die Verbindungen im angeregten Zustand ein Absorptionsmaximum im Bereich von 576-600 nm, d.h. eine blaue Anregungsfarbe, zeigen, resultieren grüne Verbindungen, die für die Verwendung in Brillengläsern in der Regel wenig geeignet sind.

In US-A-5,645,767 sowie US-A-5,869,658 wird der positive Einfluß beschrieben, den eine Indeno-Annellierung über die f-Seite des Naphthopyran-Systems auf die photochromen Eigenschaften ausübt. Die Verbindungen zeigen allgemein eine längerwellige Absorption sowie eine erhöhte Eindunklungsleistung. In EP-A-0 987 260 sowie DE 199 30 290 werden Systeme beschrieben, bei denen die Inden-Einheit zusätzlich in eine Spiro-Struktur eingebunden ist. Diese Verbindungen zeichnen sich durch exzellente Lebensdauer und schnelle Aufhellgeschwindigkeiten bei gleichzeitig hoher Eindunklungsleistung aus.

WO 97/48762 beschreibt indeno-annellierte Naphthopyranverbindungen mit bestimmten Substituenten an der 3-Stellung des Pyranringes. US-A-5,961,892 beschreibt spezifische 2H-Naphtho[1,2-b]-pyrane, 3H-Naphtho[2,1-b]pyrane sowie Indeno[2,1-f]naphtho[1,2-b]pyrane, die jeweils mindestens einen polyalkoxylierten Substituenten mit von 2 bis 50 Alkoxyeinheiten pro Substituent aufweisen.

Die Eindunklungsfarbe der im Stand der Technik beschriebenen vorgenannten Systeme ist bei 3H-Naphtho[2,1-b]pyranen vor allem von den Substituenten in 3-Stellung bzw. bei 2H-Naphtho[1,2-b]pyranen von den Substituenten in 2-Stellung abhängig. Für technische Anwendungsformen sind kosmetisch neutrale Eindunklungstöne wie braun oder grau bevorzugt. Die vorgenannten Verbindungssysteme weisen jedoch kräftige Farbtöne auf, so daß immer nur Gemische verschiedener Farbstoffe eingesetzt werden können.

Somit liegt der vorliegenden Erfindung die Aufgabe zugrunde, neue photochrome Systeme bereitzustellen, die im Vergleich zu den im Stand der Technik bislang verfügbaren Verbindungen breitere Absorptionsbanden für stumpfere Farbtöne aufweisen sollen, d.h. sie sollen insbesondere kosmetisch neutrale Eindunklungstöne zeigen. Darüber hinaus sollen diese Verbindungen eine schnelle Aufhellung bei gleichzeitig hoher Eindunklungsleistung sowie eine gute Lebensdauer aufweisen.

Diese Aufgabe wird durch die in den Ansprüchen gekennzeichneten Gegenstände gelöst. Insbesondere werden photochrome, heterocyclisch annellierte Indeno[1,2-h]-chromene mit der allgemeinen Formel (I) bereitgestellt: 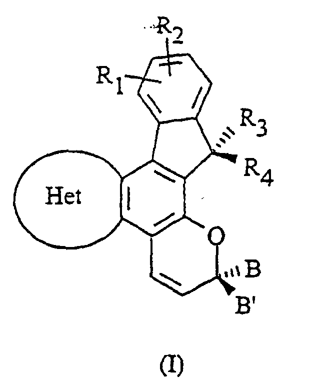 worin
die Reste R₁ und R₂ jeweils unabhängig voneinander einen Substituenten darstellen, ausgewählt aus der Gruppe A, bestehend aus Wasserstoff, Fluor, Chlor, Brom, einer Hydroxygruppe, einem (C₁-C₆)-Alkylrest, einem (C₁-C₆)-Alkoxyrest, einem (C₃-C₇)-Cycloalkylrest, der ein oder mehrere Heteroatome aufweisen kann, einem (C₁-C₆)-Acylrest, einem unsubstituierten oder monosubstituierten Phenylrest und einem unsubstituierten oder monosubstituierten Benzylrest, wobei deren Substituenten aus der Gruppe ausgewählt sind, bestehend aus einem (C₁-C₆)-Alkylrest und einem (C₁-C₆)-Alkoxyrest;
oder die Reste R₁ und R₂ zusammen einen annellierten, un-, mono- oder disubstituierten Benzo- oder Pyridoring darstellen, deren Substituenten aus der Gruppe A ausgewählt sind;
der Rest R₃ einen Substituenten darstellt, ausgewählt aus Wasserstoff, einem (C₁-C₆)-Alkylrest und -OM, wobei M ein Substituent ist, ausgewählt aus Wasserstoff, einem (C₁-C₆)-Alkylrest, einem (C₃-C₇)-Cycloalkylrest, der ein oder mehrere Heteroatome aufweisen kann, einem (C₁-C₆)-Acylrest, einem unsubstituierten oder monosubstituierten Phenylrest und einem unsubstituierten oder monosubstituierten Benzylrest, wobei deren Substituenten aus der Gruppe ausgewählt sind, bestehend aus einem (C₁-C₆)-Alkylrest und einem (C₁-C₆)-Alkoxyrest; der Rest R₄ ein Substituent ist, ausgewählt aus Wasserstoff, einem Hydroxyrest, einem (C₁-C₆)-Alkylrest, einem (C₁-C₆)-Alkoxyrest, einem (C₃-C₇)-Cycloalkylrest, einem (C₁-C₆)-Acylrest, einem jeweils un-, mono-, di- oder trisubstituiertem Phenylrest, Benzylrest, Naphthylrest, Phenanthrylrest, Pyrenylrest, Chinolylrest, Isochinolylrest, Benzofuranylrest, Thienylrest, Benzothienylrest, Dibenzofuranylrest, Dibenzothienylrest, Carbazolylrest oder Indolylrest, wobei deren Substituenten aus der Gruppe A, einem (C₁-C₆)-ω-Phenylalkylrest und einem (C₁-C₆)-ω-Phenoxyalkylrest, wobei der ω-ständige Phenylring wiederum Teil eines weiteren photochromen Pyransystems sein kann, ausgewählt sind;
oder die Reste R₃ und R₄ unter Einbeziehen des zentralen Spirokohlenstoffatoms zusammen ein gesättigtes und/oder ungesättigtes Ringglied mit 5 bis 8 Kohlenstoff-atomen darstellen, von denen maximal eines durch ein Heteroatom, ausgewählt aus der Gruppe, bestehend aus O, S und NR₅, ersetzt sein kann, wobei der Rest R₅ aus der Gruppe A ausgewählt ist, und wobei an das Ringglied mindestens ein aromatisches oder heteroaromatisches Ringsystem annelliert ist, wobei das Ringsystem ausgewählt ist aus der Gruppe E, bestehend aus Benzol, Naphthalin, Phenanthren, Pyridin, Chinolin, Furan, Thiophen, Pyrrol, Benzofuran, Benzothiophen, Indol und Carbazol, und das Ringsystem einen oder mehrere Substituenten aus der Gruppe A aufweisen kann;
der annellierte Heterocyclus (Het) eine Indoleinheit, eine Benzofuryleinheit, eine Benzothienyl, eine Thienyleinheit, eine Furyleinheit, eine Oxazolyleinheit, oder eine Imidazolyleinheit ist, wobei im Falle einer Indoleinheit, einer Benzofuryleinheit bzw. einer Benzothienyleinheit die Annellierung an das Indenochromengerüst über den 5-gliedrigen Heterocyclus erfolgt und
B und B' unabhängig voneinander aus einer der folgenden Gruppen a), b), c) oder d) ausgewählt sind, wobei sie
a) mono-, di- und trisubstituierte Arylreste sind, wobei der Arylrest Phenyl oder Naphthyl ist;
b) unsubstituierte, mono- und disubstituierte Heteroarylreste sind, wobei der He-teroarylrest Pyridyl, Furanyl, Benzofuran-2-yl, Benzofuran-3-yl, Thien-2-yl, Thien-3-yl, Benzothien-2-yl, Benzothien-3-yl, Phenazinyl, Phenoxazinyl, Phenothiazinyl oder Julolidinyl ist;
   wobei die Substituenten der Aryl- oder Heteroarylreste in a) und b) solche sind, ausgewählt aus der Gruppe, bestehend aus der vorstehend definierten Gruppe A, Hydroxy, Amino, Mono-(C₁-C₆)-alkylamino, Di-(C₁-C₆)-alkylamino, am Aromaten un-, mono- oder disubstituiertem Mono- und Diphenylamino, wobei deren Substituenten wiederum aus der Gruppe A ausgewählt sind, Pyrrolidinyl, Piperidinyl, Morpholinyl, Thiomorpholinyl, Phenazinyl, Phenoxazinyl, Phenothiazinyl, Carbazolyl, un-, mono- und disubstituiertem Pyrryl, wobei dessen Substituenten aus der Gruppe A ausgewählt sind,
c) Struktureinheiten mit den nachstehenden Strukturformeln (B) und (C) sind: 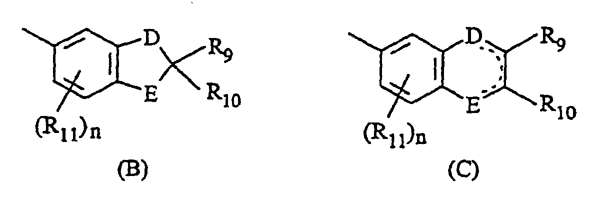 worin
   D und E unabhängig voneinander für Sauerstoff, Schwefel, Kohlenstoff oder NR₈ stehen, wobei die Reste R₈, R₉, R₁₀ und R₁₁ unabhängig voneinander einen Substituenten aus der Gruppe A darstellen, wobei n 1, 2, oder 3 ist, mit der Maßgabe, daß, wenn D in der Formel (B) NR₈ ist, E für Kohlenstoff (d.h. für Methin (=CH) oder Methylen (CH₂)) steht,
   oder
d) B und B' miteinander einen un-, mono- oder disubstituierten Fluoren-9-ylidenrest oder einen gesättigten Kohlenwasserstoffrest bilden, der C₃-C₁₂ spiro-monozyklisch, C₇-C₁₂ spiro-bizyklisch und/oder C₇-C₁₂ spiro-trizyklisch ist, wobei die Fluorensubstituenten aus der Gruppe A ausgewählt sind.

Die erfindungsgemäßen heterocyclisch annellierten Indeno [1,2-h] chromen-Derivate können als heterocyclische Verwandte von 3H-Naphto[2,1-b]pyranen betrachtet werden.

Der annellierte Heterocyclus (Het) ist eine Indoleinheit, eine Benzofuryleinheit, eine Benzothienyl, eine Thienyleinheit, eine Furyleinheit, eine Oxazolyleinheit oder eine Imidazolyleinheit, wobei im Falle einer Indoleinheit, einer Benzofuryleinheit bzw. einer Benzothienyleinheit die Annellierung an das Indenochromengerüst über den 5-gliedrigen Heterocyclus erfolgt. Besonders bevorzugt ist der annellierte Heterocyclus (Het) eine Indoleinheit, eine Benzofuryleinheit, eine Benzothienyl, eine Thienyleinheit oder eine Furyleinheit.

In einer Ausführungsform sind B und B' jeweils unabhängig voneinander mono-, dioder trisubstituierte Phenylreste, wobei die Substituenten solche sind, ausgewählt aus der Gruppe, bestehend aus der vorstehend definierten Gruppe A, Hydroxy, Amino, Mono-(C₁-C₆)-alkylamino, Di-(C₁-C₆)-alkylamino, am Aromaten un-, mono- oder disubstituiertem Mono- und Diphenylamino, wobei deren Substituenten wiederum aus der Gruppe A ausgewählt sind, Pyrrolidinyl, Piperidinyl, Morpholinyl, Thiomorpholinyl, Phenazinyl, Phenoxazinyl, Phenothiazinyl, Carbazolyl, un-, mono- und disubstituiertem Pyrryl, wobei dessen Substituenten aus der Gruppe A ausgewählt sind. In einer anderen Ausführungsform stellen B und B' jeweils unabhängig von-einander einen Julolidinylrest dar.

Besonders bevorzugte Verbindungen gemäß der vorliegenden Erfindung sind:
2-(2-Fluorphenyl)-2-phenyl-2,14-dihydro-[1]benzofuro[2,3-f]indeno[1,2-h]chromen,
2-(2-Fluorphenyl)-2-(4-methoxyphenyl)-2,14-dihydro-[1]benzofuro[2,3-f]indeno[1,2-h]-chromen,
2-(2-Fluorphenyl)-2-(4-(4-morpholinyl)phenyl)-2,14-dihydro-[1]benzofuro[2,3-f]indeno-[1,2-h]chromen,
2-(2-Fluorphenyl)-2-phenyl-2,14-dihydro-[1]benzothieno[3,2-f]indeno[1,2-h]chromen,
2-(2,4-Dimethylphenyl)-2-phenyl-2,14-dihydro-[1]benzothieno[3,2-f]indeno[1,2-h]-chromen,
2-(2-Fluorphenyl)-2-(4-methoxyphenyl)-2,14-dihydro-[1]benzothieno[3,2-f]indeno[1,2-h]-chromen,
2-(2-Fluorphenyl)-2-phenyl-2,12-dihydroindeno[1,2-h]thieno[2,3-f]chromen und
2-(2-Fluor-phenyl)-2-phenyl-9-methyl-2,9-dihydro-14H-indeno[1,2-h]indolo[3,2-f]chromen.

Fig. 1 zeigt die spektrale Transmission einer ausgewählten erfindungsgemäßen Verbindung, und zwar (2-(2-Fluorphenyl)-2-(4-methoxyphenyl)-2,14-dihydro-[1]benzofuro[2,3-f]indeno[1,2-h]chromen, wie in Beispiel 2 hergestellt) im Vergleich zu einer entsprechenden benzo-annellierten Indenochromen-Verbindung, wie in US-A-5,869,658 beschrieben.
Fig. 2 zeigt beispielhaft ein Reaktionsschema zur Herstellung erfindungsgemäßer photochromer Verbindungen.

Gemäß der vorliegenden Erfindung werden durch spezifische heterocyclische Annellierung an Indenochromen-Systeme photochrome Farbstoffe erhalten, deren photochrome Eigenschaften gegenüber den im Stand der Technik verfügbaren Verbindungen erheblich verbessert sind.

Die Eigenschaften der erfindungsgemäßen Verbindungen vom Indeno[1,2-h]chromen-Typ (I) werden im folgenden anhand einiger beispielhaft ausgewählter Verbindungen erläutert (vgl. nachstehende Tabelle 1), wobei die Erfindung selbstverständlich nicht auf diese Ausführungsformen beschränkt ist.

In Tabelle 1 werden die längstwelligen Absorptionsmaxima λₘₐₓ im eingedunkelten Zustand, die Halbwertszeit der Aufhellung t_{1/2}, d.h. die Zeit von der maximalen Eindunklung bis zu einer Transmission, die äquidistant zur maximalen Eindunklung und Aufhellung liegt, sowie die UV-Absorptions-Kante von ausgewählten erfindungsgemäßen Verbindungen aufgeführt.

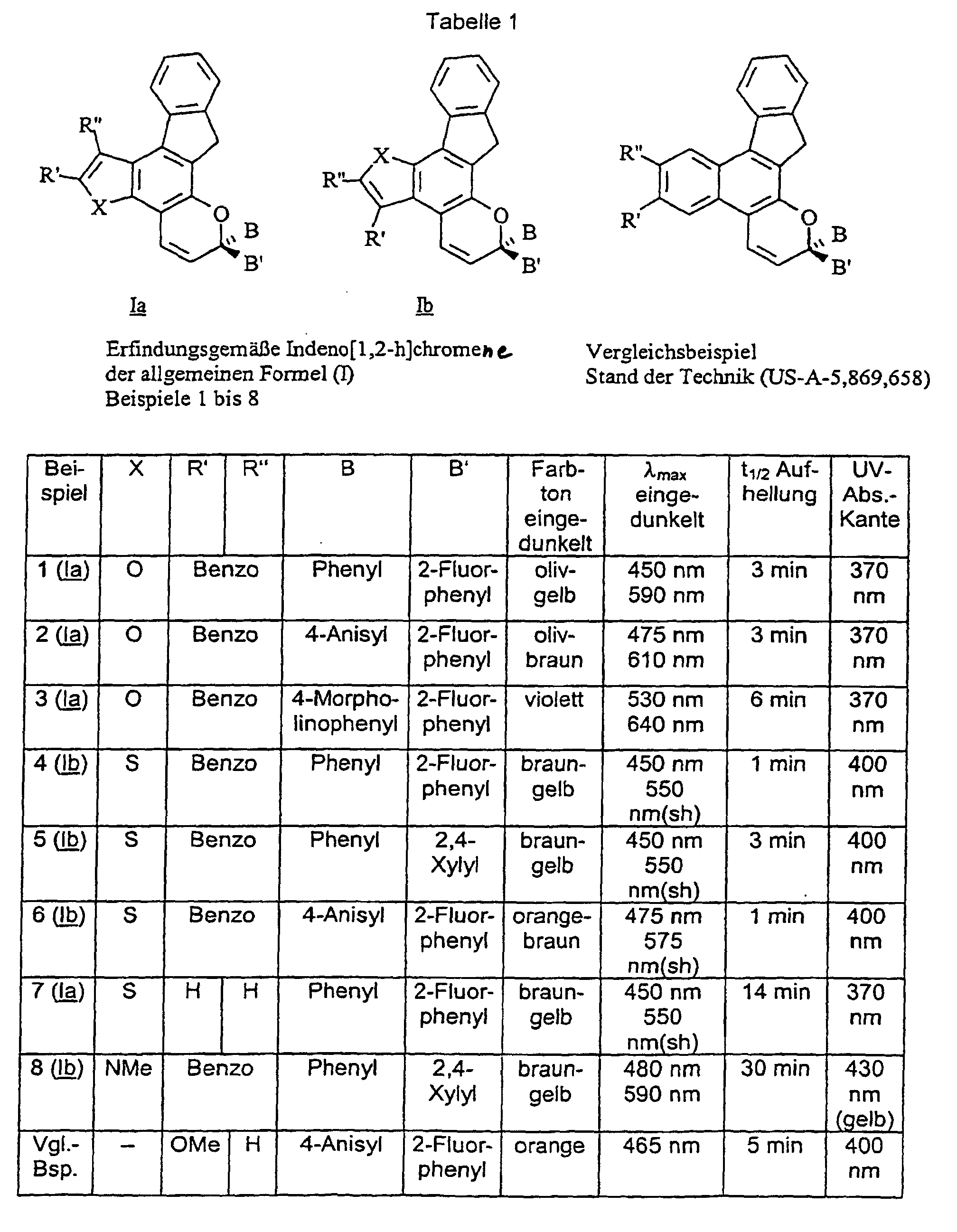

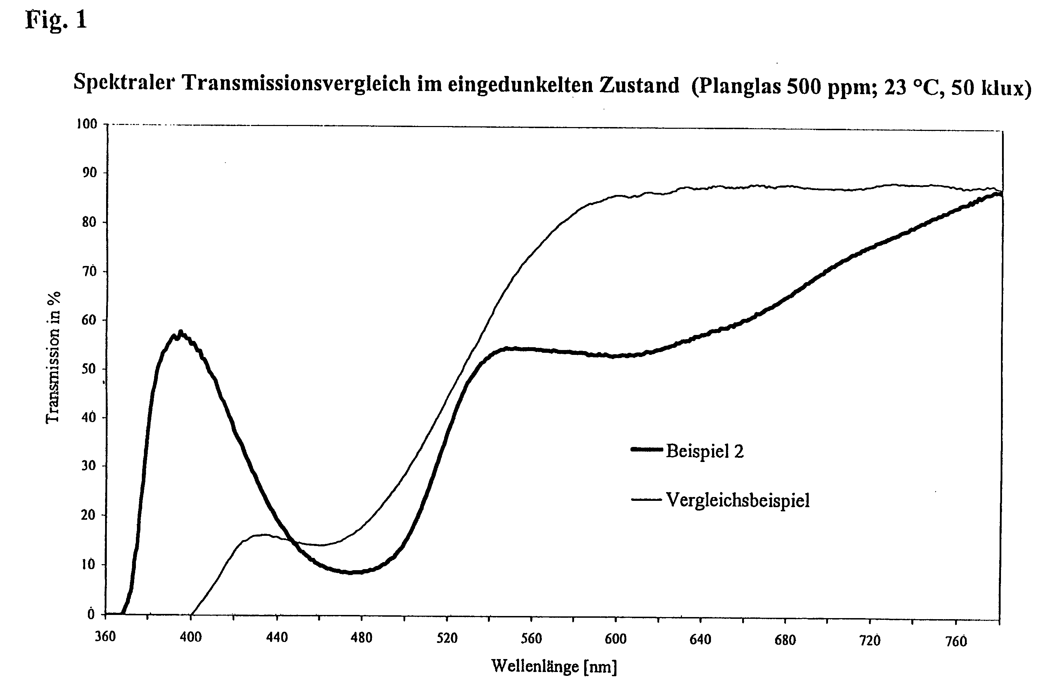

Bei den längstwelligen Absorptionsmaxima λₘₐₓ der erfindungsgemäßen Verbindungen liegen zwei diskrete Absorptionsbanden vor. Der Grund hierfür liegt in der erfindungsgemäßen heterocyclischen Annellierung des zugrundeliegenden Indenochromen-Systems. Im Gegensatz dazu weisen benzo-annellierte Indenochromen-Verbindungen, wie in US-A-5,869,658 beschrieben, nur eine Absorptionsbande auf (siehe Spektrenvergleich in Figur 1). Außerdem sind beide Banden der erfindungsgemäßen Verbindungen im Vergleich zu den benzo-annellierten Indenochromen-Verbindungen, wie in US-A-5,869,658 beschrieben, bathochrom verschoben (siehe erfindungsgemäße Beispiele 2 und 6 im Vergleich zu dem Vergleichsbeispiel mit identischen Resten B und B'). Daraus resultiert eine effizientere Abdeckung des sichtbaren Spektrums, d.h. eine höhere Lichtdämpfung gemäß der Hellempfindlichkeit des menschlichen Auges V(λ). Weiterhin sind mit den erfindungsgemäßen Verbindungen auch violette bis blauviolette Eindunklungstöne erreichbar (siehe Beispiel 3). Allgemein werden durch die beiden Absorptionsbanden stumpfe Farbtöne erzielt, was zum Erzielen kosmetisch neutraler Farbtöne vorteilhaft ist.

Bei den Halbwertszeiten t_{1/2} zeigen die erfindungsgemäßen Verbindungen eine schnellere Aufhellung als vergleichbare benzo-annellierte Indenochromen-Verbindungen, wie in US-A-5,869,658 beschrieben (siehe erfindungsgemäße Beispiele 2 und 6 im Vergleich zu dem Vergleichsbeispiel mit identischen Resten B und B'). Besonders schnell ist die Aufhellung im Falle der [1]Benzothieno[3,2-f]indeno[1,2-h]chromene (Beispiele 4-6) vom Formeltyp Ib. Bei diesen Verbindungen stört der in Richtung des photochromen Pyran-Zentrums abknickende äußere Benzol-Ring der Benzothieno-Einheit eine planare Struktur der farbigen offenen Form der photochromen Verbindung. Dadurch wird die Rückreaktion der offenen Form in die geschlossene, farblose Pyran-Verbindung beschleunigt. Der Unterschied zu den Verbindungen des Formeltyps Ia (erfindungsgemäße Beispiele 1-3 sowie 7), bei denen das relativ kleine Hetero-Atom in Richtung des photochromen Zentrums sitzt und daher die offene Form kaum beeinflussen kann, ist signifikant. Eine Ausnahme zu diesem Sachverhalt stellt Beispiel 8 dar, bei dem die stark elektronenabgebende H₃C-N Gruppe den sterischen Effekt überkompensiert. Dies ist auch an der Absorption der geschlossenen Form erkennbar, die bereits deutlich im Sichtbaren absorbiert und daher stark gelb vorgefärbt ist. Diese für normale Brillengläser eher nachteilige Eigenschaft bietet in speziellen Fällen Vorteile, wie z.B. bei Skibrillen, bei denen die gelbe Grundfärbung ein kontrastreicheres Sehen erlaubt, oder bei Brillen für Autofahrer, bei denen die weit ins Sichtbare reichende Absorption auch noch hinter der UV-Licht blockierenden Windschutzscheibe aus Verbundglas eine gute Eindunkelung ermöglicht.

Ferner ist auch die Lage der UV-Absorptions-Kanten der erfindungsgemäßen Verbindungen, d.h. die Wellenlänge, unterhalb der eine totale UV-Absorption stattfindet, von Bedeutung. Die Lage der UV-Absorptions-Kanten der erfindungsgemäßen Verbindungen variiert in Abhängigkeit vom Formeltyp. Die erfindungsgemäßen Verbindungen Ib weisen in der geschlossenen Form eine um 30 nm längerwellige Absorption als die entsprechenden Verbindungen vom Formeltyp Ia auf. Bei entsprechender Verwendung in Brillengläser bieten die Verbindungen des Formetyps Ib daher quasi bereits einen "eingebauten" UV-Schutz bis 400 nm.

Die erfindungsgemäßen Verbindungen können in Kunststoffmaterialien bzw. Kunststoffgegenständen jeglicher Art und Form für eine Vielzahl von Einsatzzwecken, für die ein photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die photochromen Indenochromen-Farbstoffe gemäß der vorliegenden Erfindung in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die Indenochromen-Farbstoffe beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Indenochromen-Farbstoffe durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfaßt beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen Indenochromen-Farbstoffe beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

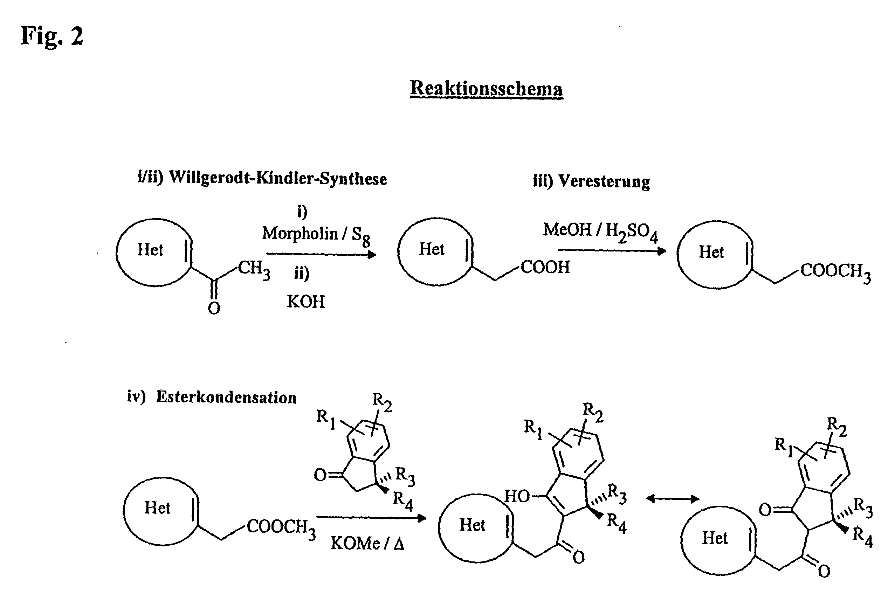

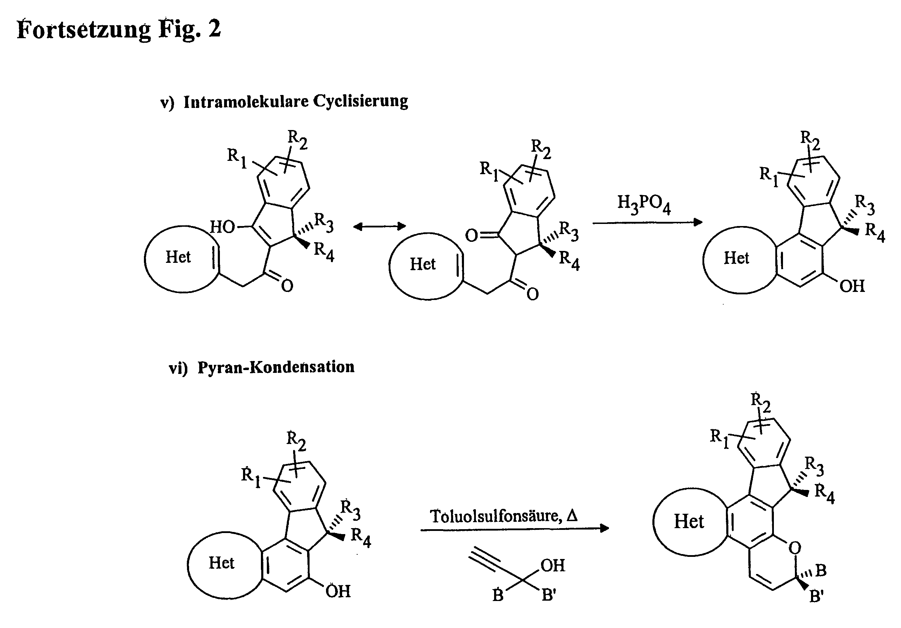

Die Figur 2 zeigt anhand eines allgemeinen Reaktionsschemas beispielhaft die Herstellung der erfindungsgemäßen Verbindungen vom Indeno[1,2-h]-chromen-Typ (I). Ausgehend von heterocyclischen Acetyl-Verbindungen werden mittels Willgerodt-Kindler-Synthesen gemäß Schritten i) und ii) die entsprechenden Essigsäure-Derivate erhalten. Nach Veresterung (Schritt iii)) erfolgt eine Esterkondensation mit 1-Indanonen (Schritt iv)) und Kaliummethylat als Base. Es entstehen 2-Acyl-1-indanon-Derivate, die als Ausgangsstoffe für die intramolekulare Cyclisierung zu heterocyclisch annellierten Fluorenol-Derivaten gemäß Schritt v) dienen. Diese Derivate werden schließlich mit 2-Propin-1-ol-Derivaten gemäß Schritt vi) zu den Indeno[1,2-h]-chromen mit der allgemeinen Formel (I) umgesetzt.

Nachfolgend wird die Herstellung beispielhaft ausgewählter, erfindungsgemäßer Indenochromen-Derivate detailliert erläutert, wobei diese Beispiele selbstverständlich den Schutzbereich der vorliegenden Erfindung nicht beschränken, sondern nur zur Veranschaulichung dienen sollen.

### BEISPIELE

### BEISPIEL 1

i) Ein Gemisch aus 2-Acetyl-1-benzofuran (32 g) und Schwefel (10 g) in 40 ml Morpholin wurde 6 h unter Rühren und Rückfluß zum Sieden erhitzt. Nach dem Abkühlen des dunklen viskosen Gemisches wurden 50 ml Ethanol zugegeben und unter Rühren im Eisbad weiter abgekühlt. Nach 10 Minuten Rühren war eine dicke Suspension entstanden, die abgesaugt wurde. Der derart erhaltene Feststoff wurde mit vorgekühltem Ethanol gewaschen, bis das Filtrat farblos war. Nach dem Trocknen wurde ein Curry-farbenes Pulver (22 g) erhalten, das mittels NMR-Spektroskopie als 4-(1-Benzofuran-2-yl-thioacetyl)morpholin identifiziert wurde.
ii) Zu einem Gemisch aus 50 g Kaliumhydroxid, 50 ml Wasser und 180 ml Ethanol wurde das in Schritt i) erhaltene Reaktionsprodukt (22 g) zugegeben. Das Reaktionsgemisch wurde 6 h unter Rühren und Rückfluß zum Sieden erhitzt. Anschließend wurde das Ethanol so weit wie möglich abdestilliert. Nach Zugabe von 300 ml Wasser zum Rückstand wurde filtriert und die bräunliche Lösung im Eisbad unter Rühren abgekühlt. Es wurde mit konz. Salzsäure angesäuert und anschließend der gebildete Niederschlag abgesaugt. Nach Waschen mit Wasser und Trocknen wurde ein gelber Feststoff (12 g) erhalten, der mittels NMR-Spektroskopie als 1-Benzofuran-2-yl-essigsäure identifiziert wurde.
iii) Das in Schritt ii) erhaltene Reaktionsprodukt (12 g) wurde unter Ausschluß von Feuchtigkeit in 200 ml Methanol und 1,5 ml konz. Schwefelsäure 6 h unter Rühren und Rückfluß zum Sieden erhitzt. Danach wurde der Ansatz in 600 ml Wasser gegossen und zweimal mit Ether extrahiert. Die vereinigten Etherphasen wurden nacheinander mit Wasser und Natriumhydrogencarbonat-Lösung gewaschen. Nach Trocknen über Natriumsulfat und Abdestillieren des Lösungsmittels wurde ein bräunliches Öl (11 g) erhalten, das mittels NMR-Spektroskopie als 1-Benzofuran-2-yl-essigsäuremethylester identifiziert wurde.
iv) Ein Gemisch des in Schritt iii) erhaltenen Reaktionsprodukts (11 g), 1-Indanon (7 g) und Kaliummethylat (6 g) in 200 ml absolutem Toluol wurde unter Ausschluß von Feuchtigkeit 4 h unter Rühren und Rückfluß zum Sieden erhitzt. Es wurde abgekühlen gelassen, 300 ml Wasser zugegeben und das Zweiphasengemisch nochmals 15 min erhitzt. Nach dem Abkühlen wurde der Ansatz in einen Scheidetrichter übergeführt und die wäßrige Phase so weit wie möglich abgetrennt. Diese wurde zur Reinigung nochmals mit Ether extrahiert und anschließend mit konz. Salzsäure angesäuert. Anschließend wurde noch zweimal mit Ether extrahiert und die vereinigten organischen Phasen nacheinander mit Wasser und Natriumhydrogencarbonat-Lösung gewaschen. Nach Trocknen über Natriumsulfat und Abdestillieren des Lösungsmittels wurde das Rohprodukt über eine Kieselgel-Säule gereinigt (Laufmittel Dichlormethan/Hexan 3:2). Die Produkteluate wurden vereinigt und das Lösungsmittel abdestilliert. Es wurde ein Feststoff (6 g) erhalten, der mittels NMR-Spektroskopie als 2-(1-Benzofuran-2-ylacetyl)-1-indanon identifiziert wurde.
v) Das in Schritt iv) erhaltene Reaktionsprodukt (5 g) wurde in 50 ml Orthophosphorsäure suspendiert und unter Rühren in ein auf 100 °C vorgeheiztes Ölbad gestellt. Nach 2 h bei 100 °C hatte sich eine bräunliche Suspension gebildet. Nach dem Abkühlen wurde das Reaktionsgemisch in 300 ml Wasser unter Rühren gegossen und die Suspension abgesaugt. Der Niederschlag wurde gründlich mit Wasser gewaschen und getrocknet. Es wurde ein hellbrauner Feststoff (4 g) erhalten, der mittels NMR-Spektroskopie als 8H-Fluoreno[3,4-b][1]benzofuran-7-ol identifiziert wurde.
vi) 1 g des in Schritt v) erhaltenen Reaktionsproduktes wurden zusammen mit 1-(2-Fluorphenyl)-1-phenyl-1-propinol (2 g; hergestellt aus 2-Fluorbenzophenon und Natriumacetylid in DMSO) in etwa 100 ml Toluol suspendiert. Nach Zusatz einer Spatelspitze 4-Toluolsulfonsäure wurde das Reaktionsgemisch 15 min unter Rühren und Rückfluß zum Sieden erhitzt. Nach kurzem Abkühlen wurde das Toluol im Vakuum abgezogen und der Rückstand in 20 ml Dichlormethan gelöst und einer Säulenchromatographie mit Aluminiumoxid (Wassergehalt 3%) als stationärer und einem Dichlormethan/Hexan-Gemisch (1:2) als mobiler Phase unterzogen. Die Produkteluate wurden vereinigt und das Lösungsmittel abdestilliert. Der Rückstand wurde bei Raumtemperatur mit wenig Ether digeriert und die Suspension nach kurzem Rühren abgesaugt. Nach Waschen mit Ether und Trocknen wurde ein leicht beigefarbener Feststoff (0,6 g) erhalten, der mittels NMR-Spektroskopie als 2-(2-Fluorphenyl)-2-phenyl-2,14-dihydro-[1]benzofuro[2,3-f]indeno[1,2-h]chromen identifiziert wurde.

### BEISPIEL 2

Die Durchführung erfolgte analog Beispiel 1, mit der Ausnahme, daß im Schritt vi) die Umsetzung mit 1-(2-Fluorphenyl)-1-(4-methoxyphenyl)-1-propinol (hergestellt aus 2-Fluor-4'-methoxybenzophenon und Natriumacetylid in DMSO) anstatt mit 1-(2-Fluorphenyl)-1-phenyl-1-propinol erfolgte. Es wurde ein leicht beigefarbener Feststoff (0,7 g) erhalten, der mittels NMR-Spektroskopie als 2-(2-Fluorphenyl)-2-(4-methoxyphenyl)-2,14-dihydro-[1]benzofuro[2,3-f]indeno[1,2-h]chromen identifiziert wurde.

### BEISPIEL 3

Die Durchführung erfolgte analog Beispiel 1, mit der Ausnahme, daß im Schritt vi) die Umsetzung mit 1-(2-Fluorphenyl)-1-(4-(4-morpholinyl)phenyl)-1-propinol (hergestellt aus 2-Fluor-4'-(4-morpholinyl)benzophenon und Natriumacetylid in DMSO) anstatt mit 1-(2-Fluorphenyl)-1-phenyl-1-propinol erfolgte. Es wurde ein farbloser Feststoff (0,7 g) erhalten, der mittels NMR-Spektroskopie als 2-(2-Fluorphenyl)-2-(4-(4-morpholinyl)phenyl)-2,14-dihydro-[1]benzofuro[2,3-f]indeno-[1,2-h]-chromen identifiziert wurde.

### BEISPIEL 4

Die Durchführung erfolgte analog Beispiel 1, mit der Ausnahme, daß im Schritt i) die Umsetzung mit 3-Acetyl-1-benzothiophen anstatt mit 2-Acetyl-1-benzofuran erfolgte. Es entstand nach Schritt v) 7H-Fluoreno[4,3-b][1]benzothiophen-6-ol und nach der Cyclisierung gemäß Schritt vi) 2-(2-Fluorphenyl)-2-phenyl-2,14-dihydro[1]benzothieno[3,2-f]indeno[1,2-h]chromen in Form eines beigefarbenen Feststoffs (0,5 g), der mittels NMR-Spektroskopie identifiziert wurde.

### BEISPIEL 5

Die Durchführung erfolgte analog Beispiel 4, mit der Ausnahme, daß im Schritt vi) die Umsetzung mit 1-(2,4-Dimethylphenyl)-1-phenyl-1-propinol (hergestellt aus 2,4-Dimethylbenzophenon und Natriumacetylid in DMSO) anstatt mit 1-(2-Fluorphenyl)-1-phenyl-1-propinol erfolgte. Es wurde ein beigefarbener Feststoff (0,7 g) erhalten, der mittels NMR-Spektroskopie als 2-(2,4-Dimethylphenyl)-2-phenyl-2,14-dihydro[1]benzothieno[3,2-f]indeno[1,2-h]-chromen identifiziert wurde.

### BEISPIEL 6

Die Durchführung erfolgte analog Beispiel 4, mit der Ausnahme, daß im Schritt vi) die Umsetzung mit 1-(2-Fluorphenyl)-1-(4-methoxyphenyl)-1-propinol (hergestellt aus 2-Fluor-4'-methoxybenzo-phenon und Natriumacetylid in DMSO) anstatt mit 1-(2-Fluorphenyl)-1-phenyl-1-propinol erfolgte Es wurde ein farbloser Feststoff (0,4 g) erhalten, der mittels NMR-Spektroskopie als 2-(2-Fluorphenyl)-2-(4-methoxyphenyl)-2,14-dihydro-[1]benzothieno[3,2-f]indeno[1,2-h]chromen identifiziert wurde.

### BEISPIEL 7

Die Durchführung erfolgte analog Beispiel 1, mit der Ausnahme, daß im Schritt i) die Umsetzung mit 2-Acetylthiophen anstatt mit 2-Acetyl-1-benzofuran erfolgte. Es entstand nach Schritt v) 6H-Fluoreno[3,4-b]thiophen-5-ol und nach der Cyclisierung gemäß Schritt vi) 2-(2-Fluorphenyl)-2-phenyl-2,12-dihydroindeno[1,2-h]thieno[2,3-f]chromen in Form eines bräunlichen Feststoffs (0,3 g), der mittels NMR-Spektroskopie identifiziert wurde.

### BEISPIEL 8

Die Durchführung erfolgte analog Beispiel 1, mit der Ausnahme, daß im Schritt i) die Umsetzung mit 3-Acetyl-1-methylindol anstatt mit 2-Acetyl-1-benzofuran erfolgte. Es entstand nach Schritt v) 12-Methyl-7H-fluoreno[4,3-b]indol-6-ol und nach der Cyclisierung gemäß Schritt vi) 2-(2-Fluor-phenyl)-2-phenyl-9-methyl-2,9-dihydro-14Hindeno[1,2-h]indolo[3,2-f]chromen in Form eines beigefarbenen Feststoffs (0,5 g), der mittels NMR-Spektroskopie identifiziert wurde.

### Herstellung der Probenkörper:

500 ppm des jeweiligen photochromen Farbstoffes wurden im verwendeten Monomer (TRANSHADE-150 der Firma Tokuyama; Brechungsindex 1,52) bei Raumtemperatur unter Rühren gelöst. Nach Zugabe eines Initiators vom Alkylperoxyester-Typ (1,5 Gew.-%) wurde zweimal entgast und der Ansatz in eine aus einem Kunststoffring und zwei Glasgießformen bestehende Form gefüllt. Anschließend wurde nach dem von der Firma Tokuyama empfohlenen Temperaturprogramm polymerisiert. Die Glasgießformen waren dabei schwarz gefärbt, damit die Gesamtheit der photochromen Farbstoffe im nicht-angeregten Zustand in die Matrix eingebaut werden kann. Nach Beendigung der Polymerisation wurden die Probenkörper noch 2 h bei 100°C getempert. Die Probenkörper waren runde Planscheiben oder Plangläser (ohne optische Wirkung ) mit ca. 2 mm Mittendicke.

### Ermittlung der Kinetikwerte sowie der längstwelligen Absorptionsmaxima:

Zur Ermittlung der Aufhellgeschwindigkeiten wurden die hergestellten Probenkörper in einer Kinetikbank PTM II der Fa. Zeiss vermessen (Bestrahlung mit 50 klux gemäß DIN EN 1836 Punkt 6.1.3.1.1.). Die Belichtungszeit betrug jeweils 15 min, die Aufhellung im Dunkeln 10 min. Die Temperatur des Glases wurde über eine thermostatisierbare Küvette geregelt. Während der Belichtung und Aufhellung wurde die Transmission - bewertet nach der Hellempfindlichkeit des menschlichen Auges V(λ) - jeweils in kurzen Zeitintervallen aufgezeichnet. Darüberhinaus liefert die PTM II-Kinetikbank zum Belichtungsende das UV/VIS-Spektrum der eingedunkelten Probenkörper, woraus die längstwelligen Absorptionsmaxima ermittelt werden können.

## Patentansprüche

1. Photochrome Indenochromene mit der allgemeinen Formel (I) : 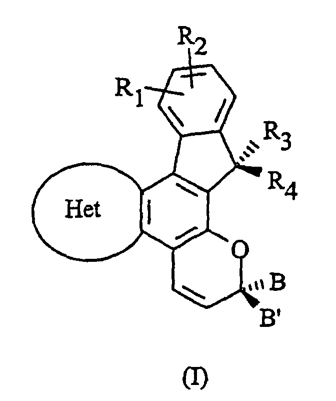 worin
die Reste R₁ und R₂ jeweils unabhängig voneinander einen Substituenten darstellen, ausgewählt aus der Gruppe A, bestehend aus Wasserstoff, Fluor, Chlor, Brom, einer Hydroxygruppe, einem (C₁-C₆)-Alkylrest, einem (C₁-C₆)-Alkoxyrest, einem (C₃-C₇)-Cycloalkylrest, der ein oder mehrere Heteroatome aufweisen kann, einem (C₁-C₆)-Acylrest, einem unsubstituierten oder monosubstituierten Phenylrest und einem unsubstituierten oder monosubstituierten Benzylrest, wobei deren Substituenten aus der Gruppe ausgewählt sind, bestehend aus einem (C₁-C₆)-Alkylrest und einem (C₁-C₆)-Alkoxyrest;
oder die Reste R₁ und R₂ zusammen einen annellierten, un-, mono- oder disubstituierten Benzo- oder Pyridoring darstellen, deren Substituenten aus der Gruppe A ausgewählt sind;
der Rest R₃ einen Substituenten darstellt, ausgewählt aus Wasserstoff, einem (C₁-C₆)-Alkylrest und -OM, wobei M ein Substituent ist, ausgewählt aus Wasserstoff, einem (C₁-C₆)-Alkylrest, einem (C₃-C₇)-Cycloalkylrest, der ein oder mehrere Heteroatome aufweisen kann, einem (C₁-C₆)-Acylrest, einem unsubstituierten oder monosubstituierten Phenylrest und einem unsubstituierten oder monosubstituierten Benzylrest, wobei deren Substituenten aus der Gruppe ausgewählt sind, bestehend aus einem (C₁-C₆)-Alkylrest und einem (C₁-C₆)-Alkoxyrest;
der Rest R₄ ein Substituent ist, ausgewählt aus Wasserstoff, einem Hydroxyrest, einem (C₁-C₆)-Alkylrest, einem (C₁-C₆)-Alkoxyrest, einem (C₃-C₇)-Cycloalkylrest, einem (C₁-C₆)-Acylrest, einem jeweils un-, mono-, di- oder trisubstituiertem Phenylrest, Benzylrest, Naphthylrest, Phenanthrylrest, Pyrenylrest, Chinolylrest, Isochinolylrest, Benzofuranylrest, Thienylrest, Benzothienylrest, Dibenzofuranylrest, Dibenzothienylrest, Carbazolylrest oder Indolylrest, wobei deren Substituenten aus der Gruppe A, einem (C₁-C₆)-ω-Phenylalkylrest und einem (C₁-C₆)-ω-Phenoxyalkylrest, wobei der ω-ständige Phenylring wiederum Teil eines weiteren photochromen Pyransystems sein kann, ausgewählt sind;
oder die Reste R₃ und R₄ unter Einbeziehen des zentralen Spirokohlenstoffatoms zusammen ein gesättigtes und/oder ungesättigtes Ringglied mit 5 bis 8 Kohlenstoff-atomen darstellen, von denen maximal eines durch ein Heteroatom, ausgewählt aus der Gruppe, bestehend aus O, S und NR₅, ersetzt sein kann, wobei der Rest R₅ aus der Gruppe A ausgewählt ist, und wobei an das Ringglied mindestens ein aromatisches oder heteroaromatisches Ringsystem annelliert ist, wobei das Ringsystem ausgewählt ist aus der Gruppe E, bestehend aus Benzol, Naphthalin, Phenanthren, Pyridin, Chinolin, Furan, Thiophen, Pyrrol, Benzofuran, Benzothiophen, Indol und Carbazol, und das Ringsystem einen oder mehrere Substituenten aus der Gruppe A aufweisen kann;
der annellierte Heterocyclus (Het) eine Indoteinheit, eine Benzofuryleinheit, eine Benzothienyl, eine Thienyleinheit, eine Furyleinheit, eine Oxazolyleinheit, oder eine Imidazolyleinheit ist, wobei im Falle einer Indoleinheit, einer Benzofuryleinheit bzw. einer Benzothienyleinheit die Annellierung an das Indenochromengerüst über den 5-gliedrigen Heterocyclus erfolgt; und
B und B' unabhängig voneinander aus einer der folgenden Gruppen a), b), c) oder d) ausgewählt sind, wobei sie
a) mono-, di- und trisubstituierte Arylreste sind, wobei der Arylrest Phenyl oder Naphthyl ist;
b) unsubstituierte, mono- und disubstituierte Heteroarylreste sind, wobei der He-teroarylrest Pyridyl, Furanyl, Benzofuran-2-yl, Benzofuran-3-yl, Thien-2-yl, Thien-3-yl, Benzothien-2-yl, Benzothien-3-yl, Phenazinyl, Phenoxazinyl, Phenothiazinyl oder Julolidinyl ist;
wobei die Substituenten der Aryl- oder Heteroarylreste in a) und b) solche sind, ausgewählt aus der Gruppe, bestehend aus der vorstehend definierten Gruppe A, Hydroxy, Amino, Mono-(C₁-C₆)-alkylamino, Di-(C₁-C₆)-alkylamino, am Aromaten un-, mono- oder disubstituiertem Mono- und Diphenylamino, wobei deren Substituenten wiederum aus der Gruppe A ausgewählt sind, Pyrrolidinyl, Piperidinyl, Morpholinyl, Thiomorpholinyl, Phenazinyl, Phenoxazinyl, Phenothiazinyl, Carbazolyl, un-, mono- und disubstituiertem Pyrryl, wobei dessen Substituenten aus der Gruppe A ausgewählt sind,
c) Struktureinheiten mit den nachstehenden Strukturformeln (B) und (C) sind: 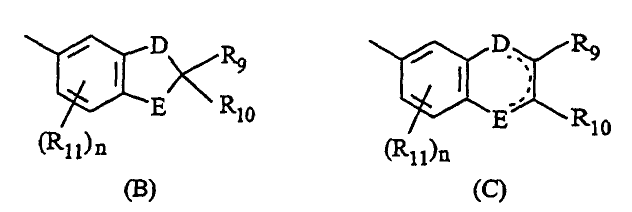 worin
D und E unabhängig voneinander für Sauerstoff, Schwefel, Kohlenstoff oder NR₈ stehen, wobei die Reste R₈, R₉, R₁₀ und R₁₁ unabhängig voneinander einen Substituenten aus der Gruppe A darstellen, wobei n 1, 2, oder 3 ist, mit der Maßgabe, daß, wenn D in der Formel (B) NR₈ ist, E für Kohlenstoff steht,
oder
d) B und B' miteinander einen un-, mono- oder disubstituierten Fluoren-9-ylidenrest oder einen gesättigten Kohlenwasserstoffrest bilden, der C₃-C₁₂ spiro-monozyklisch, C₇-C₁₂ spiro-bizyklisch und/oder C₇-C₁₂ spiro-trizyklisch ist, wobei die Fluorensubstituenten aus der Gruppe A ausgewählt sind.

2. Photochrome Indenochromene nach Anspruch 1, wobei B und B' jeweils unabhängig voneinander mono-, di- oder trisubstituierte Phenylreste sind,
wobei die Substituenten solche sind, ausgewählt aus der Gruppe, bestehend aus der vorstehend definierten Gruppe A, Hydroxy, Amino, Mono-(C₁-C₆)-alkylamino, Di-(C₁-C₆)-alkylamino, am Aromaten un-, mono- oder disubstituiertem Mono- und Diphenylamino, wobei deren Substituenten wiederum aus der Gruppe A ausgewählt sind, Pyrrolidinyl, Piperidinyl, Morpholinyl, Thiomorpholinyl, Phenazinyl, Phenoxazinyl, Phenothiazinyl, Carbazolyl, un-, mono- und disubstituiertem Pyrryl, wobei dessen Substituenten aus der Gruppe A ausgewählt sind,

3. Photochrome Indenochromene nach Anspruch 1, wobei B und B' jeweils unabhängig voneinander einen Julolidinylrest darstellen.

4. Photochrome Indenochromene nach einem der vorhergehenden Ansprüche, welche
2-(2-Fluorphenyl)-2-phenyl-2,14-dihydro-[1]benzofuro[2,3-f]indeno[1,2-h]chromen,
2-(2-Fluorphenyl)-2-(4-methoxyphenyl)-2,14-dihydro-[1]benzofuro[2,3-f]indeno[1,2-h]-chromen,
2-(2-Fluorphenyl)-2-(4-(4-morpholinyl)phenyl)-2,14-dihydro-[1]benzofuro[2,3-f]-indeno-[1,2-h]chromen,
2-(2-Fluorphenyl)-2-phenyl-2,14-dihydro-[1]benzothieno[3,2-f]indeno[1,2-h]chromen,
2-(2,4-Dimethylphenyl)-2-phenyl-2,14-dihydro-[1]benzothieno[3,2-f]indeno[1,2-h]-chromen,
2-(2-Fluorphenyl)-2-(4-methoxyphenyl)-2,14-dihydro-[1]benzothleno[3,2-f]indeno[1,2-h]-chromen,
2-(2-Fluorphenyl)-2-phenyl-2,12-dihydroindeno[1,2-h]thieno[2,3-f]chromen und
2-(2-Fluor-phenyl)-2-phenyl-9-methyl-2,9-dihydro-14H-indeno[1,2-h]indolo[3,2-f]chromen sind.

5. Verwendung der photochromen Indenochromene nach einem der Ansprüche 1 bis 4 in Kunststoffmaterialien.

6. Verwendung nach Anspruch 5, wobei das Kunststoffmaterial eine ophthalmische Linse ist.

## Claims

1. Photochromic indenochromenes having the general formula (I): 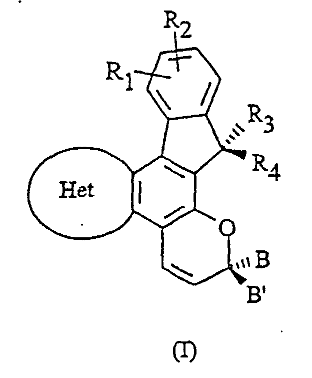 wherein
the groups R₁ and R₂ respectively represent, independently of one another, a substituent selected from the group A comprising hydrogen, fluorine, chlorine, bromine, a hydroxy group, a (C₁-C₆)-alkyl group, a (C₁-C₆)-alkoxy group, a (C₃-C₇)-cycloalkyl group which can have one or more heteroatoms, a (C₁-C₆)-acyl group, an unsubstituted or mono-substituted phenyl group and an unsubstituted or mono-substituted benzyl group, their substituents being selected from the group comprising a (C₁-C₆)-alkyl group and a (C₁-C₆)-alkoxy group;
or the groups R₁ and R₂ together represent an annellated, un-, mono- or di-substituted benzo or pyrido ring, whose substituents are selected from the group A,
the group R₃ represents a substituent selected from hydrogen, a (C₁-C₆)-alkyl group and -OM, M being a substituent which is selected from hydrogen, a (C₁-C₆)-alkyl group, a (C₃-C₇)-cycloalkyl group which can have one or more heteroatoms, a (C₁-C₆)-acyl group, an unsubstituted or mono-substituted phenyl group and an unsubstituted or mono-substituted benzyl group, their substituents being selected from the group comprising a (C₁-C₆)-alkyl group and a (C₁-C₆)-alkoxy group;
group R₄ is a substituent selected from hydrogen, a hydroxy group, a (C₁-C₆)-alkyl group, a (C₁-C₆)-alkoxy group, a (C₃-C₇)-cycloalkyl group, a (C₁₋C₆)-acyl group, a respectively un-, mono-, di- or tri-substituted phenyl, benzyl, naphthyl, phenanthryl, pyrenyl, quinolyl, isoquinolyl, benzofuranyl, thienyl, benzothienyl, dibenzofuranyl, dibenzothienyl, carbazolyl or indolyl group, their substituents being selected from the group A, a (C₁-C₆)-ω-phenylalkyl group and a (C₁-C₆)-ω-phenoxyalkyl group, the phenyl ring in ω position being capable of being in turn part of an additional photochromic pyran system;
or the groups R₃ and R₄ including the central spiro carbon atom together represent a saturated and/or unsaturated ring member having 5 to 8 carbon atoms, of which at the most one can be replaced by a heteroatom selected from the group comprising O, S and NR₅, the group R₅ being selected from the group A, and at least one aromatic or heteroaromatic ring system being annellated to the ring member, the ring system being selected from the group E, comprising benzene, naphthalene, phenanthrene, pyridine, quinoline, furan, thiophene, pyrrole, benzofuran, benzothiophene, indole and carbazole, and the ring system being able to have one or more substituents from the group A;
and
B and B' are selected independently of one another from one of the following groups a), b), c) or d), these being:
a) mono-, di- and tri-substituted aryl groups, the aryl group being phenyl or naphthyl;
b) unsubstituted, mono- and di-substituted heteroaryl groups, the heteroaryl group being pyridyl, furanyl, benzofuran-2-yl, benzofuran-3-yl, thien-2-yl, thien-3-yl, benzothien-2-yl, benzothien-3-yl, phenazinyl, phenoxazinyl, phenothiazinyl or julolidinyl;
wherein the substituents of the aryl or heteroaryl groups in a) and b) are selected from the group comprising the group A defined above, hydroxy, amino, mono-(C₁-C₆)-alkylamino, di-(C₁-C₆)-alkylamino, mono- and diphenylamino un-, mono- and di-substituted on the aromatic, their substituents being again selected from the group A, pyrrolidinyl, piperidinyl, morpholinyl, thiomorpholinyl, phenazinyl, phenoxazinyl, phenothiazinyl, carbazolyl, un-, mono- and di-substituted pyrryl, its substituents being selected from the group A,
c) structural units having the following structural formulae (B) and (C): 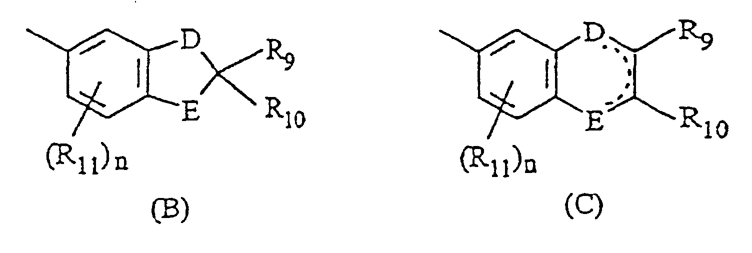 wherein
D and E independently of one another stand for oxygen, sulphur, carbon or NR₈, the groups R₈, R₉, R₁₀ and R₁₁ independently of one another representing a substituent from the group A, n being 1, 2 or 3, with the proviso that if D is NR₈ in Formula (B), E stands for carbon,
or
d) B and B' together form an un-, mono- or di-substituted fluorene-9-ylidene group or a saturated hydrocarbon group, which is C₃-C₁₂ spiro monocyclic, C₇-C₁₂ spiro bicyclic and/or C₇-C₁₂ spiro tricyclic, the fluorene substituents being selected from the group A.

2. Photochromic indenochromenes according to claim 1, wherein B and B' each independently of one another are mono-, di- or tri-substituted phenyl groups, the substituents being selected from the group comprising the group A, defined above, hydroxy, amino, mono- (C₁-C₆)-alkylamino, di-(C₁-C₆)-alkylamino, mono- and diphenylamino un-, mono- or di-substituted on the aromatic, their substituents being again selected from the group A, pyrrolidinyl, piperidinyl, morpholinyl, thiomorpholinyl, phenazinyl, phenoxazinyl, phenothiazinyl, carbazolyl, un-, mono- and di-substituted pyrryl, its substituents being selected from the group A.

3. Photochromic indenochromenes according to claim 1, wherein B and B' each independently of one another represent a julolidinyl group.

4. Photochromic indenochromenes according to one of the preceding claims which are:
2-(2-fluorophenyl)-2-phenyl-2,14-dihydro[1]benzofuro[2,3-f]indeno[1,2-h]chromene,
2-(2-fluorophenyl)-2-(4-methoxyphenyl)-2,14-dihydro[1]benzofuro[2,3-f]-indeno-[1,2-h]chromene,
2-(2-fluorophenyl)-2-(4-(4-morpholinyl)phenyl)-2,14-dihydro-[1]benzofuro[2,3-f]-indeno-[1,2-h]chromene,
2-(2-fluorophenyl)-2-phenyl-2,14-dihydro-[1]benzothieno[3,2-f]indeno[1,2-h]chromene,
2-(2,4-dimethylphenyl)-2-phenyl-2,14-dihydro[1]benzothieno[3,2-f]indeno-[1,2-h]chromene,
2-(2-fluorophehyl)-2-(4-methoxyphenyl)-2,14-dihydro[1]benzothieno[3,2-f]indeno[1,2-h]chromene,
2-(2-fluorophenyl)-2-phenyl-2,12-dihydroindeno[1,2-h]thieno[2,3-f]chromene, and
2-(2-fluorophenyl)-2-phenyl-9-methyl-2,9-dihydro-14H-indeno[1,2-h]indolo-[3,2-f]chromene.

5. Use of the photochromic indenochromenes according to one of claims 1 to 4 in plastics materials.

6. Use according to claim 5 wherein the plastics material is an ophthalmic lens.

## Revendications

1. Indénochromènes photochromes, de la formule générale (I) : 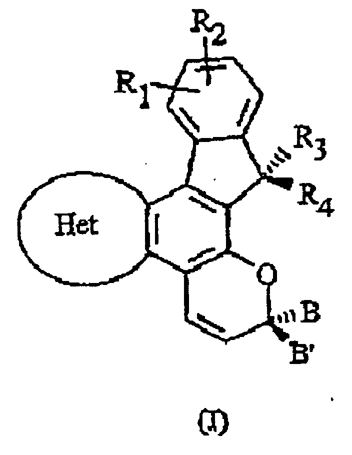 où
les restes R₁ et R₂ représentent chaque fois indépendamment, un substituant choisi parmi le groupe A, consistant en les atomes d'hydrogène, de fluor, de chlore, de brome, un radical hydroxy, un reste alkyle (C₁-C₆), un reste alcoxy (C₁-C₆), un reste cycloalkyle (C₃-C₇), qui peut présenter un ou plusieurs hétéroatomes, un reste acyle (C₁-C₆), un reste phényle non substitué ou monosubstitué, et un reste benzyle non substitué ou monosubstitué, où leurs substituants sont choisis parmi le groupe consistant en un reste alkyle (C₁-C₆) ou un reste alcoxy (C₁-C₆), ou
les restes R₁ et R₂ représentent ensemble, un cycle benzo ou pyrido, condensé, non, mono- ou disubstitué, dont les substituants sont choisis parmi le groupe A ;
le reste R₃ représente un substituant choisi parmi l'atome d'hydrogène, un reste alkyle (C₁-C₆) et -OM, où M est un substituant choisi parmi l'atome d'hydrogène, un reste alkyle (C₁-C₆), un reste cycloalkyle (C₃-C₇), qui peut présenter un ou plusieurs hétéroatomes, un reste acyle (C₁-C₆), un reste phényle non substitué ou monosubstitué, et un reste benzyle non substitué ou monosubstitué, où leurs substituants sont choisis parmi le groupe consistant en un reste alkyle (C₁-C₆) ou un reste alcoxy (C₁-C₆),
le reste R₄ est un substituant choisi parmi l'atome d'hydrogène, un reste hydroxy, un reste alkyle (C₁-C₆), un reste alcoxy (C₁-C₆), un reste cycloalkyle (C₃-C₇), un reste acyle (C₁-C₆), un reste phényle, un reste benzyle, un reste naphtyle, un reste phénanthryle, un reste pyrényle, un reste quinoléyle, un reste isoquinoléyle, un reste benzofurannyle, un reste thiényle, un reste benzothiényle, un reste dibenzofurannyle, un reste dibenzothiényle, un reste carbazoyle, un reste indolyle, chaque fois non, mono-, di- ou trisubstitué, où les substituants sont choisis parmi le groupe A, un reste ω-phénylalkyle (C₁-C₆) et un reste ω-phénylalcoxy (C₁-C₆), où le cycle phényle en position ω peut être à nouveau, une partie d'un autre système pyranne photochrome, ou les restes R₃ et R₄, avec l'intervention de l'atome de carbone spiro central, ensemble, représentent un cycle saturé et/ou insaturé, ayant 5 à 8 atomes de carbone, dont au maximum un peut être remplacé par un hétéroatome, choisi parmi le groupe consistant en O, S et NR₅, où le reste R₅ est choisi parmi le groupe A, et où sur le cycle, est condensé au moins un système cyclique aromatique ou hétéroaromatique, où le système cyclique est choisi parmi le groupe E, consistant en le benzène, le naphtalène, le phénanthrène, la pyridine, la quinoléine, le furanne, le thiophène, le pyrrole, le benzofuranne, le benzothiophène, l'indole et le carbazole, et le système cyclique peut présenter un ou plusieurs substituants parmi le groupe A ;
l'hétérocycle condensé (Het) est une unité indole, une unité benzofuryle, une unité benzothiényle, une unité thiényle, une unité furyle, une unité oxazoyle ou une unité imidazolyle, où dans le cas d'une unité indolyle, d'une unité benzofuryle ou d'une unité benzothiényle, l'annelage est réalisé sur le squelette indénochromène par l'hétérocycle à 5 membres ;
B et B' sont choisis indépendamment, parmi les groupes a), b), c) ou d) suivants, où
a) ils sont des restes aryle mono-, di- et trisubstitués, où le reste aryle est phényle ou naphtyle ;
b) ils sont des restes hétéroaryle non substitués, mono- ou disubstitués, où le reste hétéroaryle est pyridyle, furannyle, benzofurann-2-yle, benzofurann-3-yle, thièn-2-yle, thièn-3-yle, benzothièn-2-yle, benzothièn-3-yle, phénazinyle, phénoxazinyle, phénothiazinyle, ou julodinyle ;
où les substituants des restes aryle et hétéroaryle dans a) et b) sont ceux choisi parmi le groupe consistant en le groupe A défini précédemment, hydroxy, amino, monoalkyl(C₁-C₆)amino, dialkyl(C₁-C₆)amino, mono- ou diphénylamino, non, mono- ou disubstitué sur l'aromatique, où les substituants sont à nouveau choisis parmi le groupe A, pyrrolidinyle, pipéridinyle, morpholinyle, thiomorpholinyle, phénazinyle, phénoxyazinyle, phénothiazinyle, carbazolyle, pyrryle non, mono- et disubstitué, où ses substituants sont choisis parmi le groupe A,
c) ils sont les unités de structure des formules (B) et (C) suivantes : 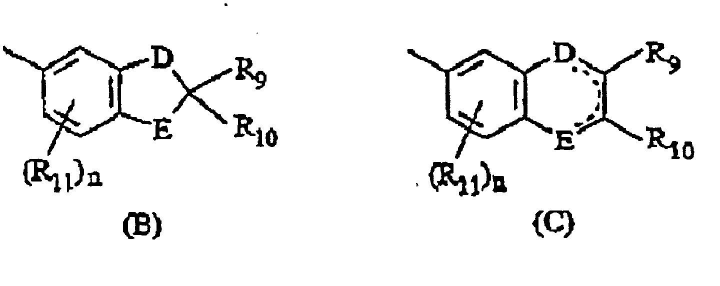 où
D et E représentent indépendamment l'un de l'autre, oxygène, soufre, carbone ou NR₈, où les restes R₈, R₉, R₁₀ et R₁₁ représentent indépendamment l'un de l'autre, un substituant parmi le groupe A, où n est 1, 2 ou 3, avec la condition que lorsque D dans la formule (B) est NR₈, E est carbone, ou
d) B et B' forment l'un avec l'autre, un reste fluorén-9-ylidène non, mono- ou disubstitué ou un reste hydrocarbure saturé, qui est spiromonocyclique en C₃-C₁₂, spirobicyclique en C₇-C₁₂ et/ou spirotricyclique en C₇-C₁₂, où les substituants du fluorène sont choisi parmi le groupe A.

2. Indénochromènes photochromes selon la revendication 1, où B et B' sont chaque fois indépendamment l'un de l'autre, un reste phényle mono-, di ou trisubstitué, où les substituants sont ceux choisis parmi le groupe consistant en le groupe A défini précédemment, hydroxy, amino, monoalkyl(C₁-C₆)amino, dialkyl(C₁-C₆)amino, mono- ou diphénylamino, non, mono- ou disubstitué sur l'aromatique, où les substituants sont à nouveau choisis parmi le groupe A, pyrrolidinyle, pipéridinyle, morpholinyle, thiomorpholinyle, phénazinyle, phénoxyazinyle, phénothiazinyle, carbazolyle, pyrryle non, mono- et disubstitué, où ses substituants sont choisis parmi le groupe A.

3. Indénochromènes photochromes selon la revendication 1, où B et B' représentent chaque fois indépendamment l'un de l'autre, un reste julodinyle.

4. Indénochromènes photochromes selon l'une des revendications précédentes, qui sont
le 2-(2-fluorophényl)-2-phényl-2,14-dihydro-[1]benzofuro[2, 3-f]indéno[1,2-h]chromène,
le 2-(2-fluorophényl)-2-(4-méthoxyphényl)-2,14-dihydro[1]benzofuro[2,3-f]indéno[1,2-h]chromène,
le 2-(2-fluorophényl)-2-(4-(4-morpholino)phényl)-2,14-dihydro-[1]benzofuro[2,3-f]indéno[1,2-h]chromène,
le 2-(2-fluorophényl)-2-phényl-2,14-dihydro-[1]benzothiéno[3,2-f]indéno[1,2-h]chromène,
le 2-(2,4-diméthylphényl)-2-phényl-2,14-dihydro-[1]benzothiéno[3,2-f]indéno[1,2-h]chromène,
le 2-(2-fluorophényl)-2-(4-méthoxyphényl)-2,14-dihydro[1]benzothiéno[2,3-f]indéno[1,2-h]chromène,
le 2-(2-fluorophényl)-2-phényl-2,12-dihydroindéno[1,2-h]thiéno[2,3-f]chromène, et
le 2-(2-fluorophényl)-2-phényl-9-méthyl-2,9-dihydro-14H-indéno[1,2-h]indolo[3, 2-f]chromène.

5. Utilisation des indénochromènes photochromes selon une des revendications 1 à 4, dans des matériaux plastiques.

6. Utilisation selon la revendication 5, où le matériau plastique est une lentille ophtalmique.
